# NEUE EUROPÄISCHE PATENTSCHRIFT

(11) **EP 1 443 073 B2**
(45) Veröffentlichungstag und Bekanntmachung des Hinweises auf die Entscheidung über den Einspruch: **05.11.2014**
(45) Hinweis auf die Patenterteilung: 16.06.2010
(21) Anmeldenummer: 03103464.8
(22) Anmeldetag: 19.09.2003
(51) Int. Cl.: C08K 3/36, C08L 77/00

(54) **Polyamidpulver mit dauerhafter, gleichbleibend guter Rieselfähigkeit**
Polyamid powder with stable, constant pourability
Poudre de polyamide avec coulabilité stable et constante

(30) Priorität: 07.11.2002 DE 10251790
(43) Veröffentlichungstag der Anmeldung: 04.08.2004
(73) Patentinhaber: Evonik Degussa GmbH, 45128 Essen (DE)
(72) Erfinder: Baumann, Franz-Erich, 48249, Dülmen (DE); Chiovaro, Johannes, 45770, Marl (DE); Monsheimer, Sylvia, 45721, Haltern am See (DE); Grebe, Maik, 44805, Bochum (DE); Christoph, Wolfgang, 45768, Marl (DE); Heinrich, Dirk, 45699, Herten (DE); Renners, Holger, 46342, Velen (DE); Scholten, Heinz, Dr., 45721, Haltern am See (DE); Schiffer, Thomas, Dr., 45721, Haltern am See (DE); Mügge, Joachim, Dr., 45721, Haltern am See (DE)

(56) Entgegenhaltungen:
- EP-A- 0 004 859
- US-A- 2 811 499
- US-A- 3 864 432

## Beschreibung

Die vorliegende Erfindung betrifft ein Verfahren zum Beschichten von Formteilen sowie ein Verfahren zur Herstellung von Probekörpern nach dem Lasersinterverfahren. Eine weitere Anwendung ist die Verwendung zur Herstellung von Kosmetika und Lacken.

Polyamidpulver finden in der Technik breite Anwendung, z.B. zum Beschichten von Metallteilen, als Additiv in Lacken und Kosmetika sowie zur Herstellung von Probekörpern oder Kleinserien nach dem Rapid-Prototyping-Verfahren durch selektives Lasersintern. An die Verarbeitbarkeit der verwendeten Pulver werden hohe Anforderungen gestellt. Ein besonders kritischer Parameter ist die Rieselfähigkeit der verwendeten Polyamidpulver. Verklumpungen in den eingesetzten Pulvern fuhren zu Inhomogenitäten der resultierenden Beschichtung oder des Probekörpers. Die eingesetzten Pulver müssen unabhängig von den Lagerbedingungen in ungeöffneten oder geöffneten Gebinden eine gute Rieselfähigkeit behalten. Speziell bei geöffneten Gebinde muss unabhängig von Witterungseinflüssen der Lagerung, wie z.B. Temperatur und Luftfeuchtigkeitsschwankungen eine gleichbleibende Verarbeitbarkeit gewährleistet sein.

Polyamidpulver nehmen über die hydrophilen Carbonsäureamidgruppen Wasser auf. Es besteht ein Zusammenhang zwischen der Kettenlänge der eingesetzten Monomere und der Wasseraufnahme des resultierenden Polyamids. So haben die Pulver auf Basis von Polyamid 11 und Polyamid 12 eine geringere Wasseraufnahme, da sie bezogen auf Methylengruppen im Polymer weniger Carbonsäureamidgruppen enthalten, als Polyamide die aus kurzkettigeren Monomeren hergestellt wurden. Die Wasseraufnahme der Polyamidpulver führt zu Verklumpungen, die eine schlechte Rieselfähigkeit des Pulvers bedingen. Die Verklumpungen sind bei Polyamidpulvern die nach dem Mahlverfahren hergestellt werden besonders ausgeprägt. Die schlechte Rieselfähigkeit geht mit einer schlechten Verarbeitbarkeit des Polyamidpulvers einher.

In der DE 29 06 647 wird ein Verfahren zur Herstellung von pulverförmigen Beschichtungsmitteln auf der Basis von Polyamiden mit mindestens 10 aliphatisch gebundenen Kohlenstoffatomen pro Carbonamidgruppe beschrieben. Die nach diesem Verfahren hergestellten Beschichtungsmittel lassen sich zur Herstellung lackähnlicher Überzüge auf Metallen einsetzen. Die Beschichtung kann nach dem Wirbelsinterverfahren, dem Flammspritzverfahren oder dem elektrostatischen Beschichtungsverfahren erfolgen. Die nach diesem Verfahren hergestellten Beschichtungszusammensetzungen bilden bei Erhitzen auf Temperaturen oberhalb der Filmbildungstemperatur, ohne Erzeugen von störendem Qualm, Überzüge mit einer glatten Oberfläche, guter Kantenbeschichtung, guter Elastizität und ausgezeichneter Beständigkeit gegenüber alkalischen wässrigen Lösungen. Die in DE 29 06 647 hergestellten Beschichtungspulver weisen eine definierte Korngrößenverteilung auf, die durch die Herstellungsbedingungen vorgegeben wird. Die Rieselfähigkeit der so hergestellten Polyamide ist sofort nach der Herstellung als ausreichend zu erachten. Wie bei allen Polyamidpulvern des Standes der Technik tritt auch bei nach diesem Verfahren hergestellten Pulvern nach einer Lagerung bei variierender Luftfeuchtigkeit und Temperatur eine Beeinträchtigung der Rieselfähigkeit durch Klumpenbildung ein.

In der DE 31 13 392 wird ein Verfahren zum Vermindern der elektrostatischen Aufladung von thermoplastischen pulverförmigen Polyamiden offenbart. Das nach diesem Verfahren hergestellte Beschichtungspulver aus thermoplastischen Kunststoffen enthält neben thermoplastischen pulverförmigen Polyamiden einen Zusatz von geringen Mengen eines hochdispersen anorganischen Pulvers, wobei das hochdisperse, anorganische Pulver mit einem antistatischen Mittel beschichtet ist. Das anstistatische Mittel sind organische Ammonium- oder Aminverbindungen. Von dem so beschichteten Pulver werden 0,01 bis 0,03 Gewichtsteile, bezogen auf 100 Gewichtsteile Polyamid zugesetzt. Das so ausgerüstete Polyamidpulver zeigt gegenüber einem nicht ausgerüsteten Pulver eine verbesserte Wirbelbarkeit im Wirbelsinterprozess. Jedoch sind in DE 31 13 392 keine Angaben enthalten, ob das beschriebene Beschichtungspulver nach Lagerung bei variierender Temperatur und Luftfeuchtigkeit seine Rieselfähigkeit und somit seine Eignung als Beschichtungspulver behält. Durch die hydrophile Beschichtung der antistatischen Mittel ist anzunehmen, dass die Rieselfähigkeit der Beschichtungszusammensetzung bei Lagerung bei variierender Temperatur oder Luftfeuchtigkeit abnimmt.

Petrovicova et al. [J. Appl. Polym. Sc. 77 (2000) 1684-1699; ibid. 78 (2000) 2272-2289] beschreibt den high velocity oxy-fuel process (HVOF-Prozess). Bei dem beschriebenen HVOF-Prozess wird ein Polymerpulver mit Pigmenten zur Verbesserung der Eigenschaften der Beschichtungsschicht versehen und zur Beschichtung von Metallteilen verwendet. Das Polymerpulver wird mittels einer Verbrennungs-Spritzpistole geschmolzen und im geschmolzenen Zustand auf den zu beschichtenden Werkstoff aufgebracht. Petrovicova untersuchte den Einfluß von hydrophilen Kieselsäuren, hydrophoben Kieselsäuren, Ruß oder A 1100 γ-Aminopropyltriethoxysilan modifiziertem Silika als Pigment in der Mischung mit Nylon 11 auf die Eigenschaften der resultierende Beschichtung. Hierzu wurden jeweils 5, 10, 15 und 20 Volumen-Prozent der Pigmente zum Nylon 11 gegeben. Dies entspricht einem Anteil von 0,3 bis 3 Gewichtsprozent Pigment in der Beschichtungszusammensetzung. Das jeweilige Pigment und Nylon 11 werden in einer Mühle, die Zirconia-Kugeln als Mahlhilfe enthält, 48 h gemischt und vermahlen. Durch die lange Mahlzeit wird das Pigment sehr homogen in die Nylon 11-Körner eingearbeitet. Petrovicova fand heraus, dass die Eigenschaften der resultierenden Beschichtung von dem Anteil an Pigment abhängig sind. Die höchsten Festigkeitseigenschaften der resultierenden Beschichtung werden mit 20 Volumenprozent Pigment in der Beschichtungszusammensetzung erreicht. In beiden Dokumenten wird nicht beschrieben, dass die eingesetzten Pigmente die Rieselfähigkeit der Beschichtungszusammensetzung verbessern. Der Fachmann erhält auch keinen Hinweis auf den Einfluss der offenbarten Pigmente auf die Rieselfähigkeit der Beschichtungszusammensetzung.

Die technische Aufgabe der vorliegenden Erfindung ist es, Verfahren und Verwendungen umfassend eine Zusammensetzung enthaltend ein Polyamid, Derivate von Polyamiden oder Mischungen derselben bereitzustellen, die unabhängig von den Lagerbedingungen der Zusammensetzung eine gleichbleibende Rieselfähigkeit und somit Verarbeitbarkeit gewährleistet. Die Zusammensetzung soll unabhängig von den Lagerbedingungen, insbesondere bei Temperatur- oder Luftfeuchtigkeitsschwankungen, mit gleichbleibender Qualität zum Beschichten von Formteilen, zur Herstellung von Probekörpern nach dem Lasersinterverfahren, zur Herstellung von Kosmetika und zur Herstellung von Lacken verwendet werden können.

Die technische Aufgabe wird gelöst durch die Lehre der unabhängigen Ansprüche, wobei eine Zusammensetzung enthaltend 88 - 99,99 Gewichtsprozent eines Polymers, ausgewählt aus der Gruppe Polyamide, Derivate von Polyamiden oder Mischungen derselben und 0,01 bis 0,25 Gewichtsprozent einer Rieselhilfe ausgewählt aus der Gruppe hydrophobierte Kieselsäure, hydrophobe Kieselsäure oder Mischungen derselben, wobei der nach ISO 787/2 bestimmte Trocknungsverlust der Rieselhilfe nach 5 Tagen Konditionierung bei einer relativen Luftfeuchtigkeit von 95 % kleiner oder gleich 1 % ist, zur Verfügung gestellt wird. In der Zusammensetzung können optional noch 0 bis 11,99 Gewichtsprozent eines oder mehrerer üblicher Additive enthalten sein.

Unerwarterweise zeigen so ausgewählte Rieselhilfen in Kombination mit von Natur aus hydrophilen Polyamiden eine von den Lagerbedingungen unabhängige gute Rieselfähigkeit. Eine Rieselhilfe mit einem Trocknungsverlust kleiner oder gleich 1 % nach 5 Tagen Konditionierung bei einer relativen Luftfeuchtigkeit von 95 % kann selbst kein oder nur sehr wenig Wasser oder Wasserdampf aus der Umgebung aufnehmen. Die Rieselhilfe kann daher in der Zusammensetzung nicht als Trocknungsmittel wirken und daher die Wasseraufnahme des Polyamids nicht verringern. Der Fachmann würde annehmen, dass in der Zusammensetzung nun das Polyamid Wasser oder Wasserdampf aus der Umgebungsluft aufnimmt und deshalb verklumpt. Diese zu erwartende Verklumpung des Polyamides wird überraschenderweise bei der Zusammensetzung nicht beobachtet.

Es ist bevorzugt, dass das Polymer ausgewählt wird aus der Gruppe Polyamid 12, Polyamid 11, Polyamid 6.10, Polyamid 6.12, Polyamid 10.12, Polyamid 6, Polyamid 6.6 oder Mischungen derselben. Vorzugsweise sind 95 bis 99,99 Gew.-%, insbesondere 97 bis 99,99 Gew.-%, weiter bevorzugt 99,0 bis 99,99 noch weiter bevorzugt 99,5 bis 99,99, insbesondere 99,75 bis 99,99 und besonders bevorzugt 99,8 bis 99,99 Gew.-% des Polymers in der Zusammensetzung enthalten. In einer weiteren bevorzugten Ausführungsform sind 99,82 bis 99,99 Gew.-%, insbesondere 99,85 - 99,99 Gew.-%, weiter bevorzugt 99,88 bis 99,99 Gew.-% und besonders bevorzugt 99,9 bis 99,99 Gew.-% des Polymers in der Zusammensetzung enthalten.

Die eingesetzten Rieselhilfen werden in einer bevorzugten Ausführungsform ausgewählt aus der Gruppe, hydrophobierte Kieselsäure, hydrophobe Kieselsäure oder Mischungen derselben. Vorzugsweise sind 0,01 bis 0,2, insbesondere 0,01 bis 0,18 Gew.-%, weiter bevorzugt 0,01 bis 0,15, noch weiter bevorzugt 0,01 bis 0,12 und besonders bevorzugt 0,01 bis 0,1 Gew.-% der Rieselhilfe in der Zusammensetzung enthalten.

Die hydrophobe oder hydrophobierte Kieselsäure wird vorzugsweise durch Umsetzung von Kieselsäuren mit Hydrophobierungsmitteln erhalten. Die Hydrophobierung erfolgt vorzugsweise nach Fällung oder Pyrolyse der Kieselsäure und weitgehende Umsetzung freier OH-Gruppen der Kieselsäure mit z.B. Silanen, Silazanen oder Siloxanen. Bevorzugte Hydrophobierungsmittel sind Hexadecylsilan, Dimethyldichlorsilan, Hexamethyldisilazan, Octamethylcyclotetrasiloxan, Polydimethylsiloxan oder Methacrylsilane

Das eingesetzte Polymer hat eine mittlere Teilchengröße d50 von 0,1 bis 250 µm, bevorzugt 1 bis 150 µm, besonders bevorzugt 3 bis 120 µm, und weiter bevorzugt 5 bis 80 µm.

Rieselhilfen bestehen in der Regel aus nanoskaligen Primärpartikeln. So weisen beispielsweise handelsübliche Aerosile eine Primärpartikelgröße von einigen Nanometern (z. B. 7 bis 12 nm) auf. Die Primärpartikel bilden üblicherweise größere Agglomerate oder Aggregate aus. Beim Einmischen der Rieselhilfe im Polymer-Pulver werden die Agglomerate oder Aggregate nur teilweise aufgebrochen, so dass neben einzelnen nanoskaligen Primärteilchen vor allem Agglomerate und Aggregate die Funktion der Rieselhilfe wahrnehmen. Bei der Bestimmung der mittleren Teilchengröße der Rieselhilfe werden üblicherweise sowohl einzelne Primärpartikel als auch Aggregate und Agglomerate gefunden.

Die erfindungsgemäße Rieselhilfe hat eine mittlere Teilchengröße von 5 nm bis 200 µm. Es ist bevorzugt, dass die Rieselhilfe eine mittlere Teilchengröße von 10 nm bis 150 µm, besonders bevorzugt 100 nm bis 100 µm hat.

Die eingesetzte Rieselhilfe hat eine spezifische Oberfläche von 20 bis 600 m²/g. Es ist bevorzugt, dass die Rieselhilfe eine spezifische Oberfläche von 40 bis 550 m²/g, besonders bevorzugt 60 bis 500 m²/g, und noch weiter bevorzugt 60 bis 450 m²/g hat.

Das optional eingesetzte Additiv, das keine Rieselhilfe ist, ist ein Additiv, das üblicherweise eingesetzt wird. Es kann ausgewählt werden aus der Gruppe enthaltend Metallpulver, Ferrite, organisches Buntpigment, anorganisches Buntpigment, Ruß, unbunter organischer Füllstoff, unbunter anorganischer Füllstoff, amorpher oder teilkristalliner Füllstoff, unterkühlte Schmelzen, pH-Wert Regler oder Mischungen derselben. In einer bevorzugten Ausführungsform ist das Additiv Aluminiumpulver, Bariumsulfat, Titandioxid, Zirkoniumdioxid, Glasfasern, Glaskugeln oder Mineralfasern. Die Additive dienen in einer bevorzugten Ausfuhrungsfbrm der weiteren Modifikation der Polyamidpulver wie zum Beispiel dem Einfärben oder der Optimierung der mechanischen Eigenschaften der mit dem Polyamidpulver hergestellten Beschichtung oder Probekörpers. Es kann ein Additiv oder eine Mischung mehrere Additive in der Zusammensetzung enthalten sein. In der Zusammensetzung sind in einer bevorzugten Ausführungsform 0 bis 4,99 Gew.-%, weiter bevorzugt 0 bis 2,99 Gew.-%, insbesondere 0 bis 0,99 Gew.-%, weiter bevorzugt 0 bis 0,49 Gew.-%, insbesondere 0 bis 0,249 Gew.-% und besonders bevorzugt 0 bis 0,19 Gew.-% des Additivs enthalten. In einer weiteren bevorzugten Ausführungsform sind 0 bis 0,17 Gew.-%, insbesondere 0 bis 0,14 Gew.-%, weiter bevorzugt 0 bis 0,11 Gew.-% und besonders bevorzugt 0 bis 0,09 Gew.-% des Additivs enthalten.

Die Veränderung der Rieseldauer von Polyamidpulvern nach Lagerung ist ein Indiz, ob sich die Rieselfähigkeit der Pulver durch die Lagerung verändert hat. Wenn die Rieselfähigkeit der Polyamidpulver durch Lagerung infolge von z.B. Temperatur- und Luftfeuchtigkeitsschwankungen abgenommen hat, wird die Rieseldauer zunehmen. Die Lagerung von Polyamidpulvern unter Temperatur- und Luftfeuchtigkeitschwankungen kann im Labor simuliert werden und die Rieseldauer kann gemäß DIN 53492 bestimmt werden.

Die Polyamidpulver zeigen nach einer Konditionierung bei 95 % relativer Luftfeuchtigkeit (6 Tage, 40 °C) und nachfolgender Konditionierung bei 50 % relativer Luftfeuchtigkeit (24 Stunden, 20 °C) eine Zunahme der Rieseldauer, die nach DIN 53492 bestimmt wird, von weniger als 20 %. In einer weiteren bevorzugten Ausführungsform beträgt die Zunahme der Rieseldauer weniger als 10 %, weiter bevorzugt weniger als 5 %.

Der Trocknungsverlust der Rieselhilfe kann nach ISO 787/2 bestimmt werden. Hierzu wird die Rieselhilfe 5 Tage bei einer relativen Luftfeuchtigkeit von 95 % konditioniert. Es ist bevorzugt, dass die Konditionierung bei 20 °C und unter Normaldruck durchgeführt wird. Nach 5 Tagen Konditionierung wird der Trocknungsverlust der Rieselhilfe entsprechend ISO 787/2 durch Erhitzen für 2 Stunden auf 105°C bestimmt. Der Trocknungsverlust wird als Änderung der Masse der Rieselhilfe in Prozent angegeben. Es ist bevorzugt, dass der Trocknungsverlust kleiner 0,8 %, besonders bevorzugt kleiner 0,6 %, weiter bevorzugt kleiner 0,5 % und noch weiter bevorzugt kleiner 0,4 % ist.

Grundsätzlich sind alle Arten von Mischern geeignet um das Polyamidpulver herzustellen. Die Mischdauer liegt vorzugsweise bei weniger als 120 Minuten, weiter bevorzugt bei weniger als 90 Minuten. In einer bevorzugten Ausführungsform werden das Polyamidpulver und die Rieselhilfe in einem Schnellmischer gemischt. Es kann jeder Schnellmischer verwendet werden, der eine hohe Scherung während der Mischung ermöglicht. Die Mischdauer liegt im Bereich von weniger als 10 Minuten. Es ist bevorzugt, das die Mischdauer weniger als fünf Minuten beträgt. Der Vorteil dieser bevorzugten Ausführungsform liegt in einer innigen und homogenen Vermischung von Polyamidpulver und Rieselhilfe ohne dass die Rieselhilfe in das Polyamidkorn gedrückt wird.

Die Zusammensetzung kann in Verfahren zum Beschichten von Formteilen eingesetzt werden. Die Formteile können aus Metall oder einer Metalllegierung bestehen. Bei dem Beschichtungsverfahren kann die Zusammensetzung während des Beschichtungsvorganges durch Einblasen eines Gases fluidisiert werden. Alternativ kann die Zusammensetzung mittels einer Sprühpistole fein zerstäubt werden. Die verwendeten Verfahren können das Wirbelsinterverfahren, Rotationssinterverfahren, elektrostatisches Beschichten, das Tribobeschichten oder das Minicoat-Verfahren sein. Da die Zusammensetzung gut rieselt und keine Verklumpungen aufweist bilden sich homogene Schichten auf den Formteilen aus.

Überraschenderweise zeigt es sich, dass bei der Verwendung der Zusammensetzung beim Wirbelsinterverfahren das sogenannte Phänomen der Kraterbildung auf der entstehenden Polyamidoberfläche vermieden wird. Eine Erklärung dieses Phänomens, ohne jedoch eine Theorie hierfür zu liefern, könnte sein, dass die Rieselhilfen nur gering oder kein Wasser im Vergleich zu den Rieselhilfen des Standes der Technik aufnehmen. Somit kann während des Beschichtungsvorganges kein Wasser aus der entstehenden Schicht verdampfen und somit eine Kraterbildung bedingen.

Beim Aufbau von Probekörpern nach dem Lasersinterverfahren werden Pulver schichtweise aufgetragen und jeweils unter Einwirkung von Laserlicht zu einem homogenen Körper verschmolzen. Agglomerate im Pulver und/oder eine unzureichende Rieselfähigkeit des Pulvers können zu ungleichmäßigen Pulverschichten in der jeweiligen Bauebene führen und bedingen am fertigen Bauteil Hohlräume sowie Störungen an den Kanten und Oberflächen. Durch ein agglomeratfreies Pulver mit gleichbleibend guter Rieselfähigkeit bilden sich unter den Bedingungen des Lasersinterprozesses nur wenige und/oder kleine Hohlräume im Probekörper aus, wodurch insbesondere die mechanischen Eigenschaften des Bauteils verbessert werden. Gleichzeitig wird die Anzahl an Fehlstellen auf der Oberfläche und den Kanten minimiert. Die Oberfläche des Probekörpers wird homogen ausgeformt. Es zeigte sich, dass die Zusammensetzung auch bei der Herstellung von Probekörpern nach dem Lasersinterverfahren eine verbesserte Qualität der Probekörper bedingt. Dadurch, dass die Zusammensetzung gleichbleibend rieselfähig ist, sind keine störenden Verklumpungen und somit keine größeren Hohlräume in ihr vorhanden.

Die Zusammensetzung kann auch zur Herstellung von Kosmetika verwendet werden. Hierbei zeigt sich, dass die gleichbleibend gute Rieselfähigkeit zu einer sehr homogenen Mischung der einzelnen Bestandteile der Kosmetika fährt. Verklumpungen, die in Zusammensetzungen des Standes der Technik nach Lagerung bei unterschiedlicher Temperatur und Luftfeuchtigkeit vorhanden sind, führen bei dem Einsatz zur Herstellung von Kosmetika zu Agglomeraten in den Kosmetika, die diese in ihrer Qualität nachteilig beeinflussen.

Die Zusammensetzung kann auch zur Herstellung von Lacken verwendet werden. Ähnlich wie bei Kosmetika ist es bei Lacken notwendig, dass die Qualität der einzelnen Komponenten von den Lagerbedingungen unabhängig ist. Es treten keine Agglomerate in den Lacken auf, die in nicht gewünschtem Maße die Oberfläche des aufgetragenen Lackes markieren, mattieren oder auftauen.
Die vorliegende Erfmdung wird an den nachfolgenden Beispielen erläutert.

### Beispiele

### Beispiel 1

Die Rieselhilfe Aerosil® R 812 und Aerosil® R 972 und das Vergleichsbeispiel Aerosil® 200 werden 5 Tage lang bei einer Temperatur von 20 °C und einer relativen Luftfeuchtigkeit von 95 % konditioniert. Anschließend wird der Trocknungsverlust gemäß ISO 787/2 nach Lagerung für 2 Stunden bei 105 °C bestimmt. Der Trocknungsverlust wird als Masseänderung der Rieselhilfen durch Trocknung in Prozent angegeben.

**Tabelle 1:**

| Rieselhilfe | Trocknungsverlust [%] | Bemerkungen |
|---|---|---|
| Aerosil® R 812* | < 0,1 | keine Verklumpungen |
| Aerosil® R 972* | < 0,1 | keine Verklumpungen |
| Aerosil® 200* (Vergleichsbeispiel) | 18,8 | klumpiges Pulver |

| | | |
|---|---|---|
| *Aerosil® ist ein eingetragenes Warenzeichen der Degussa AG | | |

Die gemessenen Trocknungsverluste (Tabelle 1) geben wieder, dass die Rieselhilfen Aerosil® R 812 und Aerosil® R 972 bei Lagerung in einer Umgebung mit hoher rel. Luftfeuchtigkeit kein Wasser (Trocknungsverlust < 0,1 %) aufnehmen und nicht verklumpen. Das Vergleichsbeispiel Aerosil® 200 nimmt beim Lagerung in einer Umgebung, die eine sehr hohe Luftfeuchte besitzt, so viel Wasser auf, dass das vorher rieselfähige Pulver verklumpt.

### Beispiel 2

Zu 2 kg (100 Teile) Polyamid-12-Pulver, hergestellt gemäß DE 29 06 647 mit einem mittleren Korndurchmesser d₅₀ von 98 µm (Laserbeugung) und einer Schüttdichte gemäß DIN 53466 von 480 g/l, wird 5 g (0,25 Teile) Aerosil®R 812 im Dry-Blend-Verfahren unter Benutzung eines Henschel-Mischers P10 bei 400 U/min 5 Minuten gemischt. Die Rieselfähigkeit der erhaltenen Zusammensetzung wird gemäß DIN 53492 unmittelbar nach dem Mischen bestimmt. Nach einer 6-tägigen Konditionierung bei 40 °C und 95 % rel. Luftfeuchtigkeit und nachfolgender Konditionierung der Zusammensetzung für 24 Stunden bei 20 °C und 50 % rel. Luftfeuchtigkeit wird die Rieselfähigkeit erneut bestimmt. Die Rieselfähigkeit der Zusammensetzung vor und nach Konditionierung wird durch Bestimmung der Rieseldauer in Sekunden für 150 g Zusammensetzung gemäß DIN 53492 ermittelt. Die Rieseldauer ist in Tabelle 2 wiedergegeben.

### Beispiel 3

2 kg (100 Teile) des Polyamid-12-Pulvers aus Beispiel 2 und 5 g (0,25 Teile) Aerosil® R 972 werden gemäß Beispiel 2 gemischt. Die Rieselfähigkeit der Zusammensetzung wird, wie in Beispiel 2 beschrieben, bestimmt und ist in Tabelle 2 aufgefährt.

### Beispiel 4

2 kg (100 Teile) des Polyamid-12-Pulvers aus Beispiel 2 und 0,5 g (0,025 Teile) Aerosil® R 812 werden gemäß Beispiel 2 gemischt. Die Rieselfähigkeit der Zusammensetzung wird, wie in Beispiel 2 beschrieben, bestimmt und ist in Tabelle 2 aufgefährt.

### Beispiel 5

2 kg (100 Teile) des Polyamid-12-Pulvers aus Beispiel 2 und 0,3 g (0,015 Teile) Aerosil® R 812 werden gemäß Beispiel 2 gemischt. Die Rieselfähigkeit der Zusammensetzung wird, wie in Beispiel 2 beschrieben, bestimmt und ist in Tabelle 2 aufgefährt.

### Beispiel 6

2 kg (100 Teile) des Polyamid-12-Pulvers aus Beispiel 2 und 5 g (0,25 Teile) Aerosil® 200 werden, wie in Beispiel 2 beschrieben, gemischt. Die Rieselfähigkeit der erhaltenen Zusammensetzung wird, wie in Beispiel 2 beschrieben, bestimmt und ist in Tabelle 2 aufgeführt.

**Tabelle 2**

| Zusammensetzung | Rieseldauer nach Mischen | Rieseldauer nach Konditionierung bei erhöhter Luftfeuchtigkeit | Prozentuale Änderung der Rieseldauer durch Konditionierung bei erhöhter Luftfeuchtigkeit |
|---|---|---|---|
| Zusammensetzung aus Beispiel 2 | 12,8 s | 13,2 s | +3 % |
| Zusammensetzung aus Beispiel 3 | 13,1 s | 14,2 s | +8 % |
| Zusammensetzung aus Beispiel 4 | 13,4 s | 13,9 s | +4 % |
| Zusammensetzung aus Beispiel 5 | 15,6 s | 17,2 s | +10 % |
| Zusammensetzung aus Beispiel 6 (Vergleich) | 12,6 s | 18,1 s | +44 % |

| | | | |
|---|---|---|---|
| Die Rieseldauer der Zusammensetzung wurde für 150 g der Zusammensetzung gemäß DIN 53492 bestimmt. | | | |

Die Zusammensetzungen aus Beispiel 2 bis 5 zeigen nach einer Konditionierung von 6 Tagen in einer Klimakammer bei 40 °C und einer relativen Luftfeuchtigkeit von 95 % und anschließender Konditionierung für 24 Stunden bei 20 °C und 50 % rel. Luftfeuchtigkeit keine signifikante Änderung der Rieselfähigkeit. Die Zunahme der Rieseldauer liegt zwischen 3 % und 10 %. Beding durch die gleichbleibend gute Rieselfähigkeit, unabhängig von den Lagerbedingungen der Zusammensetzungen aus Beispielen 2 bis 5, ist keine Beeinträchtigung in der Verarbeitbarkeit der Zusammensetzungen aufgetreten. Das Vergleichsbeispiel 6 zeigt im frisch gemischten Zustand eine Rieselfähigkeit, die mit den Beispielen 2 bis 5 vergleichbar ist. Das Vergleichsbeispiel 6 zeigt aber nach einer Konditionierung von 6 Tagen in einer Klimakammer bei 40 °C und 95 % relativer Feuchte und anschließender Konditionierung für 24 Stunden bei 20 °C und 50 % rel. Luftfeuchtigkeit eine signifikante Zunahme der Rieseldauer um 44 % und somit eine signifikante Verschlechterung der Rieselfähigkeit. Diese Verschlechterung der Rieselfähigkeit führte zu Verarbeitungsschwierigkeiten und Qualitätsproblemen der mit dieser Zusammensetzung hergestellten Produkte.

## Patentansprüche

1. Verfahren zum Beschichten von Formteilen, wobei das Formteil mit einer Zusammensetzung beschichtet wird, wobei die Zusammensetzung enthält
88 bis 99.99 Gew.-% eines Polymers ausgewählt aus der Gruppe Polyamide, Derivate von Polyamiden oder Mischungen derselben und
0.01 bis 0,25 Gew.% einer Rieselhilfe, ausgewählt aus der Gruppe hydrophobierte Kieselsäure, hydrophobe Kieselsäure oder Mischungen derselben, wobei der nach ISO 787/2 bestimmte Trocknungsverlust der Rieselhilfe nach 5 Tagen Konditionierung bei einer relativen Luftfeuchtigkeit von 95 % kleiner oder gleich 1 % ist.

2. Verfahren nach Anspruch 1,
**dadurch gekennzeichnet,**
**dass** das Polymer ausgewählt wird aus der Gruppe Polyamid 12, Polyamid 11, Polyamid 6.10, Polyamid 6.12, Polyamid 10.12, Polyamid 6, Polyamid 6.6 oder Mischungen derselben.

3. Verfahren nach Anspruch 1 oder 2,
**dadurch gekennzeichnet,**
**dass** das Polymer eine mittlere Teilchengröße d50 von 0,1 bis 250 µm hat.

4. Verfahren nach Anspruch 1,
**dadurch gekennzeichnet,**
**dass** die Rieselhilfe eine spezifische Oberfläche von 20 bis 600 qm/g hat.

5. Verfahren nach Anspruch 1 oder 4,
**dadurch gekennzeichnet,**
**dass** die Rieselhilfe eine mittlere Teilchengröße von 10 nm bis 150 µm hat.

6. Verfahren nach einem oder mehreren der vorhergehenden Ansprüche,
**dadurch gekennzeichnet,**
**dass** die Zusammensetzung nach sechs Tagen Konditionierung bei 95 % relativer Luftfeuchtigkeit und 40 °C und nachfolgender Konditionierung bei 50 % relativer Luftfeuchtigkeit für 24 Stunden bei 20 °C eine Zunahme der gemäß DIN 53492 bestimmten Rieseldauer von weniger als 20 % aufweist.

7. Verfahren nach einem der Ansprüche 1 bis 6,
**dadurch gekennzeichnet,**
**dass** das Formteil aus einem Metall oder aus einer Metallegierung besteht.

8. Verfahren nach einem der Ansprüche 1 bis 6,
**dadurch gekennzeichnet,**
**dass** die Zusammensetzung während des Beschichtungsvorganges durch Einblasen eines Gases fluidisiert wird.

9. Verfahren nach einem der Ansprüche 1 bis 6,
**dadurch gekennzeichnet,**
**dass** das Verfahren das Wirbelsintern, Rotationssintern, elektrostatische Beschichten, Tribo-Beschichten oder Minicoat-Verfahren ist.

10. Verfahren zur Herstellung von Probekörpern, wobei die Probekörper aus einer Zusammensetzung im Lasersinter-Verfahren hergestellt werden, wobei die Zusammensetzung enthält
88 bis 99,99 Gew.-% eines Polymers ausgewählt aus der Gruppe Polyamide, Derivate von Polyamiden oder Mischungen derselben und
0,01 bis 0,25 Gew.-% einer Rieselhilfe, ausgewählt aus der Gruppe hydrophobierte Kieselsäure, hydrophobe Kieselsäure oder Mischungen derselben, wobei der nach ISO 787/2 bestimmte Trocknungsverlust der Rieselhilfe nach 5 Tagen Konditionierung bei einer relativen Luftfeuchtigkeit von 95 % kleiner oder gleich 1 % ist.

11. Verfahren nach Anspruch 10, **dadurch gekennzeichnet, dass** das Polymer ausgewählt wird aus der Gruppe Polyamid 12, Polyamid 11, Polyamid 6.10, Polyamid 6.12, Polyamid 10.12, Polyamid 6, Polyamid 6.6 oder Mischungen derselben.

12. Verwendung einer Zusammensetzung, welche enthält
88 bis 99,99 Gew.-% eines Polymers ausgewählt aus der Gruppe Polyamide, Derivate von Polyamiden oder Mischungen derselben und
0,01 bis 0,25 Gew.-% einer Rieselhilfe, ausgewählt aus der Gruppe hydrophobierte Kieselsäure, hydrophobe Kieselsäure oder Mischungen derselben, wobei der nach ISO 787/2 bestimmte Trocknungsverlust der Rieselhilfe nach 5 Tagen Konditionierung bei einer relativen Luftfeuchtigkeit von 95 % kleiner oder gleich 1 % ist,
**dadurch gekennzeichnet,**
**dass** die Zusammensetzung zum Wirbelsintern, Rotationssintern, elektrostatischen Beschichten, Tribo-Beschichten oder im Minicoat-Verfahren verwendet wird.

13. Verwendung einer Zusammensetzung, welche enthält
88 bis 99,99 Gew.-% eines Polymers ausgewählt aus der Gruppe Polyamide, Derivate von Polyamiden oder Mischungen derselben und
0,01 bis 0,25 Gew.% einer Rieselhilfe, ausgewählt aus der Gruppe hydrophobierte Kieselsäure, hydrophobe Kieselsäure oder Mischungen derselben, wobei der nach ISO 787/2 bestimmte Trocknungsverlust der Rieselhilfe nach 5 Tagen Konditionierung bei einer relativen Luftfeuchtigkeit von 95 % kleiner oder gleich 1 % ist
**dadurch gekennzeichnet,**
**dass** die Zusammensetzung zur Herstellung von Kosmetika verwendet wird.

14. Verwendung einer Zusammensetzung, welche enthält
88 bis 99,99 Gew.-% eines Polymers ausgewählt aus der Gruppe Polyamide, Derivate von Polyamiden oder Mischungen derselben und
0,01 bis 0,25 Gew.% einer Rieselhilfe, ausgewählt aus der Gruppe hydrophobierte Kieselsäure, hydrophobe Kieselsäure oder Mischungen derselben, wobei der nach ISO 787/2 bestimmte Trocknungsverlust der Rieselhilfe nach 5 Tagen Konditionierung bei einer relativen Luftfeuchtigkeit von 95 % kleiner oder gleich 1 % ist
**dadurch gekennzeichnet,**
**dass** die Zusammensetzung zur Herstellung von Lacken verwendet wird.

## Claims

1. Process for the coating of moldings, where the molding is coated with a composition, wherein the composition comprises
from 88 to 99.99% by weight of a polymer selected from the group polyamides, derivatives of polyamides, and mixtures of these, and
from 0.01 to 0.25% by weight of a flow aid, selected from the group hydrophobicized silica, hydrophobic silica, and mixtures of these, where the drying loss from the flow aid after 5 days of conditioning at relative humidity of 95% is less than or equal to 1%, determined to ISO 787/2.

2. Process according to Claim 1,
**characterized in that**
the polymer is selected from the group nylon-12, nylon-11, nylon-6,10, nylon-6,12, nylon-10,12, nylon-6, nylon-6,6, and mixtures of these.

3. Process according to Claim 1 or 2,
**characterized in that**
the polymer has a median particle size d50 of from 0.1 to 250 µm.

4. Process according to Claim 1,
**characterized in that**
the flow aid has a specific surface area of from 20 to 600 m²/g.

5. Process according to Claim 1 or 4,
**characterized in that**
the flow aid has a median particle size of from 10 nm to 150 µm.

6. Process according to one or more of the preceding claims,
**characterized in that**
the increase in the flow time of the composition after six days of conditioning at 95% relative humidity and 40°C followed by conditioning at 50% relative humidity for 24 hours at 20°C is less than 20% to DIN 53492.

7. Process according to any of Claims 1 to 6,
**characterized in that**
the molding is composed of a metal or of a metal alloy.

8. Process according to any of Claims 1 to 6,
**characterized in that**
the composition is fluidized during the coating procedure by injection of a gas.

9. Process according to any of Claims 1 to 6,
**characterized in that**
the process is fluidized-bed sintering, rotational sintering, electrostatic coating, tribocoating or the minicoat process.

10. Process for producing test specimens, where the test specimens are produced from a composition by the laser-sintering process, wherein the composition comprises
from 88 to 99.99% by weight of a polymer selected from the group polyamides, derivatives of polyamides, and mixtures of these, and
from 0.01 to 0.25% by weight of a flow aid, selected from the group hydrophobicized silica, hydrophobic silica, and mixtures of these, where the drying loss from the flow aid after 5 days of conditioning at relative humidity of 95% is less than or equal to 1%, determined to ISO 787/2.

11. Process according to Claim 10, **characterized in that** the polymer is selected from the group of nylon-12, nylon-11, nylon-6,10, nylon-6,12, nylon-10,12, nylon-6, nylon-6,6, and mixtures of these.

12. Use of a composition which comprises
from 88 to 99.99% by weight of a polymer selected from the group polyamides, derivatives of polyamides, and mixtures of these, and
from 0.01 to 0.25% by weight of a flow aid, selected from the group hydrophobicized silica, hydrophobic silica, and mixtures of these, where the drying loss from the flow aid after 5 days of conditioning at relative humidity of 95% is less than or equal to 1%, determined to ISO 787/2,
**characterized in that**
the composition is used for fluidized-bed sintering, rotational sintering, electrostatic coating or tribocoating or in the minicoat process.

13. Use of a composition which comprises
from 88 to 99.99% by weight of a polymer selected from the group polyamides, derivatives of polyamides, and mixtures of these, and
from 0.01 to 0.25% by weight of a flow aid, selected from the group hydrophobicized silica, hydrophobic silica, and mixtures of these, where the drying loss from the flow aid after 5 days of conditioning at relative humidity of 95% is less than or equal to 1%, determined to ISO 787/2,
**characterized in that**
the composition is used for producing cosmetics.

14. Use of a composition which comprises from 88 to 99.99% by weight of a polymer selected from the group polyamides, derivatives of polyamides, and mixtures of these, and
from 0.01 to 0.25% by weight of a flow aid, selected from the group hydrophobicized silica, hydrophobic silica, and mixtures of these, where the drying loss from the flow aid after 5 days of conditioning at relative humidity of 95% is less than or equal to 1%, determined to ISO 787/2,
**characterized in that**
the composition is used for producing coating materials.

## Revendications

1. Procédé pour le revêtement de pièces façonnées, où la pièce façonnée est revêtue par une composition, la composition contenant
- 88 à 99,99% en poids d'un polymère choisi dans le groupe des polyamides, des dérivés de polyamides ou leurs mélanges et
- 0,01 à 0,25% en poids d'un adjuvant d'écoulement, choisi dans le groupe de silices hydrofugées, des silices hydrophobes ou leurs mélanges, où la perte au séchage déterminée selon la norme ISO 787/2 de l'adjuvant d'écoulement après un conditionnement de 5 jours à une humidité relative de l'air de 95% est inférieure ou égale à 1%.

2. Procédé selon la revendication 1, **caractérisé en ce que** le polymère est choisi dans le groupe formé par le polyamide 12, le polyamide 11, le polyamide 6.10, le polyamide 6.12, le polyamide 10.12, le polyamide 6, le polyamide 6.6 ou leurs mélanges.

3. Procédé selon la revendication 1 ou 2, **caractérisé en ce que** le polymère présente une grosseur moyenne des particules d50 de 0,1 à 250 µm.

4. Procédé selon la revendication 1, **caractérisé en ce que** l'adjuvant d'écoulement présente une surface spécifique de 20 à 600 m²/g.

5. Procédé selon la revendication 1 ou 4, **caractérisé en ce que** l'adjuvant d'écoulement présente une grosseur moyenne des particules de 10 nm à 150 µm.

6. Procédé selon l'une ou plusieurs des revendications précédentes, **caractérisé en ce que** la composition, après un conditionnement pendant six jours à une humidité relative de l'air de 95% et à 40°C et un conditionnement consécutif à une humidité relative de l'air de 50% pendant 24 heures à 20°C présente une augmentation de la durée d'écoulement déterminée selon la norme DIN 53492 inférieure à 20%.

7. Procédé selon l'une des revendications 1 à 6, **caractérisé en ce que** la pièce façonnée est constituée par un métal ou un alliage métallique.

8. Procédé selon l'une des revendications 1 à 6, **caractérisé en ce que** la composition est fluidisée en insufflant un gaz pendant le procédé de revêtement.

9. Procédé selon l'une des revendications 1 à 6, **caractérisé en ce que** le procédé est le frittage par tourbillonnement, le frittage par rotation, le revêtement électrostatique, le revêtement tribologique ou le procédé de "Minicoat".

10. Procédé pour la préparation d'éprouvettes, où les éprouvettes sont préparées à partir d'une composition dans le procédé de frittage au laser, la composition contenant
- 88 à 99,99% en poids d'un polymère choisi dans le groupe des polyamides, des dérivés de polyamides ou leurs mélanges et
- 0,01 à 0,25% en poids d'un adjuvant d'écoulement, choisi dans le groupe de silices hydrofugées, des silices hydrophobes ou leurs mélanges, où la perte au séchage déterminée selon la norme ISO 787/2 de l'adjuvant d'écoulement après un conditionnement de 5 jours à une humidité relative de l'air de 95% est inférieure ou égale à 1%.

11. Procédé selon la revendication 10, **caractérisé en ce que** le polymère est choisi dans le groupe formé par le polyamide 12, le polyamide 11, le polyamide 6.10, lepolyamide 6.12, le polyamide 10.12, le polyamide 6, le polyamide 6.6 ou leurs mélanges.

12. Utilisation d'une composition qui contient
- 88 à 99,99% en poids d'un polymère choisi dans le groupe des polyamides, des dérivés de polyamides ou leurs mélanges et
- 0,01 à 0,25% en poids d'un adjuvant d'écoulement, choisi dans le groupe de silices hydrofugées, des silices hydrophobes ou leurs mélanges, où la perte au séchage déterminée selon la norme ISO 787/2 de l'adjuvant d'écoulement après un conditionnement de 5 jours à une humidité relative de l'air de 95% est inférieure ou égale à 1%,
**caractérisée en ce que** la composition est utilisée pour le frittage par tourbillonnement, le frittage par rotation, le revêtement électrostatique, le revêtement tribologique ou dans le procédé de "Minicoat".

13. Utilisation d'une composition qui contient
- 88 à 99,99% en poids d'un polymère choisi dans le groupe des polyamides, des dérivés de polyamides ou leurs mélanges et
- 0,01 à 0,25% en poids d'un adjuvant d'écoulement, choisi dans le groupe de silices hydrofugées, des silices hydrophobes ou leurs mélanges, où la perte au séchage déterminée selon la norme ISO 787/2 de l'adjuvant d'écoulement après un conditionnement de 5 jours à une humidité relative de l'air de 95% est inférieure ou égale à 1%,
**caractérisée en ce que** la composition est utilisée pour la préparation de produits cosmétiques.

14. Utilisation d'une composition qui contient
- 88 à 99,99% en poids d'un polymère choisi dans le groupe des polyamides, des dérivés de polyamides ou leurs mélanges et
- 0,01 à 0,25% en poids d'un adjuvant d'écoulement, choisi dans le groupe de silices hydrofugées, des silices hydrophobes ou leurs mélanges, où la perte au séchage déterminée selon la norme ISO 787/2 de l'adjuvant d'écoulement après un conditionnement de 5 jours à une humidité relative de l'air de 95% est inférieure ou égale à 1%,
**caractérisée en ce que** la composition est utilisée pour la préparation de laques.
